# EUROPEAN PATENT SPECIFICATION

(11) **EP 1 767 165 B1**
(45) Date of publication and mention of the grant of the patent: **01.03.2017**
(21) Application number: 05021025.1
(22) Date of filing: 27.09.2005
(51) Int. Cl.: A61B 18/14, F28F 21/02

(54) **Cooled ablation needle**
Gekühlter Ablationsnadel
Une aiguille refroidie pour ablation

(43) Date of publication of application: 28.03.2007
(73) Proprietor: Covidien AG, 8212 Neuhausen am Rheinfall (CH)
(72) Inventor: Peterson, Darion, Boulder CO 80301 (US)
(74) Representative: Soames, Candida Jane

(56) References cited:
- US-A- 4 074 718
- US-A- 6 074 389
- US-A1- 2005 155 743
- US-B1- 6 287 305

## Description

### BACKGROUND

### Technical Field

The present disclosure relates to advances in medical systems and procedures for prolonging and improving human life and, more particularly, to novel electrosurgical instruments for tissue ablation, and systems for tissue ablation including the electrosurgical instruments.

### Discussion of Related Art

Therapeutic lesions in living bodies have been accomplished for many decades using radio-frequency (RF) and other forms of energy. The procedures have been particularly useful in the field of neurosurgery, typically where RF ablation electrodes (usually of elongated cylindrical geometry) are inserted into a living body. A typical form of such ablation electrodes incorporates an insulated sheath from which an exposed (uninsulated) tip extends.

Generally, the ablation electrode is coupled between a grounded RF power source, e.g., an electrosurgical generator, (outside the body) and a reference ground or indifferent electrode, e.g., return electrode, for contacting a large surface of the body. When an RF voltage is provided between the ablation electrode and the reference ground, RF current flows from the ablation electrode through the body. Typically, the current density is very high near the tip of the ablation electrode, which heats and destroys the adjacent tissue.

In the past, RF ablation electrodes have incorporated temperature sensors, for example, in the form of a thermistor or thermocouple as disclosed in U.S. Pat. No. 4,411,266 to Cosman. Typically, the sensor is connected to a monitoring apparatus for indicating temperature to assist in accomplishing a desired lesion. As generally known, for a given tip geometry and tip temperature, lesions of a prescribed size can be made quite consistently, also disclosed in U.S. Pat. No. 4,411,266 to Cosman.

Over the years, a wide variety of RF electrode shapes and configurations have been used, for example, several current forms are available from Radionics, Inc., located in Burlington, Mass. Such electrodes have been used to accomplish lesions in a wide variety of targets within the body, including the brain, the spinal column and the heart.

An important criterion when using electrode ablation systems relates to the temperature of the tip achieved during the ablation process. Specifically, it is desirable to maintain the temperature of certain ablation electrodes, of a given tip geometry, below 100° C. At a temperature at or above 100° C, the tissue surrounding the ablation electrode will tend to boil and char. Consequently, the lesion size for a given electrode geometry generally has been considered to be somewhat limited by the fact that the tissue near the tip must not exceed 100° C.

Essentially, during RF ablation, the electrode temperature is highest near the tip, because the current density is the highest at that location. Accordingly, temperature falls off as a function of distance from the electrode tip and, except for possible abnormalities in tissue conductivity and so on, in a somewhat predictable and even calculable pattern. As an attendant consequence, the size of RF lesions for a given electrode geometry have been somewhat limited.

One proposed solution to the limitation of lesion's size has been to employ "off-axis" electrodes, for example the so called Zervas Hypophysectomy Electrode or the Gildenberg Side-Outlet electrode, as manufactured by Radionics, Inc., Burlington, Mass. However, such systems, in requiring multiple tissue punctures, increase trauma to the patient.

Considering lesion size, it has been seen that lesions in the brain of up to 10 to 12 millimeters, by using very large ablation electrodes, may be produced. However, in order to produce similarly sized lesions or larger sized lesions with relatively smaller ablation electrodes, ablations systems including ablation electrodes with conduits which deliver cooling fluid to the tip thereof have been developed. Reference may be made to U.S. Patents 5,951,546; 6,506,189; 6,530,922; and 6, 575,969, the entire contents of each of which being incorporated herein by reference, for a detailed discussion of such systems. Generally, ablation electrodes with cooled conductive tips produce larger lesion volumes as compared to ablation tips which are not cooled.

Accordingly, a need exists for electrosurgical instruments for tissue ablation, systems for tissue ablation including the electrosurgical instruments, and method for ablating tissues containing abnormalities such as cancerous tumors using the systems for tissue ablation.

US 6,074,389 discloses a cooled electrosurgical system including an electrosurgical device having at least one electrode for applying electrical energy to tissue to cut the tissue or coagulate it.In one version, the electrode includes an internal cavity in which a cooling medium such as water is contained. The internal cavity is closed at the distal end of the device such that the cooling medium is contained within the electrode at the surgical site such that the cooling medium does not contact the tissue being treated. In another version the electrode is a heat pipe having at one end air cooled fins.

### SUMMARY

According to an aspect of the present disclosure, there is provided an ablation electrode assembly operatively connectable to a source of electrosurgical energy and to a source of cooling fluid, the ablation electrode assembly comprising: a hub defining a chamber therein; at least one electrically conductive ablation needle extending from the hub, the needle being cylindrical and defining cavity therein, wherein the cavity of the ablation needle extends to the distal end portion of the cavity, and the ablation needle including a distal end portion configured to penetrate tissue, said distal end portion of the needle being electrically and thermally conductive for establishing electric and thermal communication with the tissue; a heat sink fabricated from an anisotropic material disposed within the cavity operatively connected to the ablation needle, wherein a distal end of the heat sink is in conductive engagement with a distal end surface of the cavity of the ablation needle, the heat sink being connected to the ablation needle to draw energy away from at least the distal end portion thereof, the heat sink including a proximal end extending into the chamber of the hub; an inlet conduit fluidly connected to the hub for delivering fluid into the chamber thereof from the source of fluid, wherein the fluid withdraws energy from the proximal end of the heat sink, an outlet conduit fluidly connected to the chamber of the hub for delivering fluid from the chamber thereof, , such that fluid enters the chamber of the hub through the inlet conduit and exits the chamber of the hub through the outlet conduit, whereby fluid contacts and washes over or across the proximal end of the heat sink and withdraws heat and/or energy therefrom and, in turn, from the ablation needle.

The heat sink is fabricated from a conductive material which is anisotropic, such as, for example, a graphite fiber.

The ablation assembly may further include an insulative coating surrounding at least a portion of a length of the ablation needle. The distal end portion of the ablation needle may be exposed.

According to another aspect of the present disclosure there is provided an ablation electrode assembly operatively connectable to a source of electrosurgical energy and to a source of cooling fluid, the ablation electrode assembly comprising: a hub defining a chamber therein; at least one electrically conductive ablation needle) extending from the hub, the ablation needle including a distal end portion configured to penetrate tissue, said distal end portion being electrically and thermally conductive for establishing electric and thermal communication with the tissue; a heat sink which encases at least a portion of a length of the ablation needle to draw energy away from at least the distal end portion thereof, the heat sink including a proximal end extending into the chamber of the hub; an inlet conduit fluidly connected to the hub for delivering fluid into the chamber thereof from the source of fluid, wherein the fluid withdraws energy from the proximal end of the heat sink, an outlet conduit fluidly connected to the chamber of the hub for delivering fluid from the chamber thereof such that fluid enters the chamber of the hub through the inlet conduit and exits the chamber of the hub through the outlet conduit, whereby fluid contacts and washes over or across the proximal end of the heat sink and withdraws heat and/or energy therefrom and, in turn, from the ablation needle.

In the assembly in which the heat sink encases at least a portion of a length of the ablation needle, desirably, the distal end portion of the ablation needle is exposed. In an embodiment, an insulative coating may surround at least a portion of a length of the heat sink encasing the ablation needle.

The assembly may further include a source or electrosurgical energy electrically connected to the ablation needle. The ablation system may still further include a source of cooling fluid fluidly connected to the chamber of the hub. The ablation system may further include a thermal-sensing circuit electrically connected to the ablation needle for measuring a temperature of the ablation needle. The ablation system may further include a microprocessor connected to and for coordinating operation of the source of electrosurgical energy and the source of fluid.

In an embodiment, it is envisioned that the ablation needle is solid. It is envisioned that a plurality of ablation needles may be provided.

According to a further aspect of the present disclosure, an ablation electrode assembly operatively connectable to a source of electrosurgical energy and to a source of cooling fluid is provided. The ablation electrode assembly includes a hub defining a chamber therein; at least one electrically conductive ablation needle extending from the hub, the ablation needle including a distal end portion configured to penetrate tissue, said distal end portion being electrically and thermally conductive for establishing electric and thermal communication with the tissue; a heat sink operatively connected to the ablation needle, the heat sink being connected to the ablation needle to draw energy away from at least the distal end portion thereof, the heat sink including a proximal end extending into the chamber of the hub; and a conduit fluidly connected to the hub for delivering fluid into the chamber thereof from the source of fluid, wherein the fluid withdraws energy from the proximal end of the heat sink.

The heat sink is fabricated from a conductive material including an anisotropic material, such as, for example, a graphite fiber.

The ablation electrode assembly further includes an outlet conduit fluidly connected to the chamber of the hub for delivering fluid from the chamber thereof.

The ablation needle may define a cavity therein. The heat sink may be disposed within the cavity of the ablation needle. The cavity of the ablation needle may extend to the distal end portion thereof. Accordingly, a distal end of the heat sink may be in conductive engagement with a distal end surface of the cavity of the ablation needle.

The ablation electrode may further include an insulative coating surrounding at least a portion of a length of the ablation needle. The distal end portion of the ablation needle desirably remains exposed.

In an embodiment, it is envisioned that the heat sink encases at least a portion of a length of the ablation needle. In this embodiment, desirably, the distal end portion of the ablation needle remains exposed. It is envisioned that an insulative coating may surround at least a portion of a length of the heat sink encasing the ablation needle.

The ablation electrode assembly may further include a thermal-sensing circuit electrically connected to the ablation needle for measuring a temperature of the ablation needle.

The ablation needle may be solid. It is envisioned that a plurality of ablation needles may be provided.

### BRIEF DESCRIPTION OF THE DRAWINGS

Further features and advantages of the invention will become readily apparent from the following specification and from the drawings, in which:
FIG. 1 is a partial cross-sectional view of a prior art cooled needle electrode;
FIG. 2 is a broken-away partial cross-sectional view of the tip part of the cooled needle electrode of FIG. 1;
FIG. 3 is a schematic, partial cross-sectional illustration, of an ablation system in accordance with an embodiment of the present disclosure;
FIG. 4 is a schematic, partial cross-sectional illustration, of an embodiment of an ablation electrode assembly of the ablation system of FIG. 3;
FIG. 5 is a schematic, partial cross-sectional illustration, of another embodiment of an ablation electrode assembly of the ablation system of FIG. 3;
FIG. 6 is a schematic, partial cross-sectional illustration, of yet another embodiment of an ablation electrode assembly of the ablation system of FIG. 3;
FIG. 7 is a schematic, partial cross-sectional illustration, of still another embodiment of an ablation electrode assembly of the ablation system of FIG. 3;
FIG. 8 is a schematic perspective view of an ablation system according to another embodiment of the present disclosure; and
FIG. 9 is a schematic longitudinal cross-sectional view of the ablation system of FIG. 8.

### DETAILED DESCRIPTION OF EMBODIMENTS

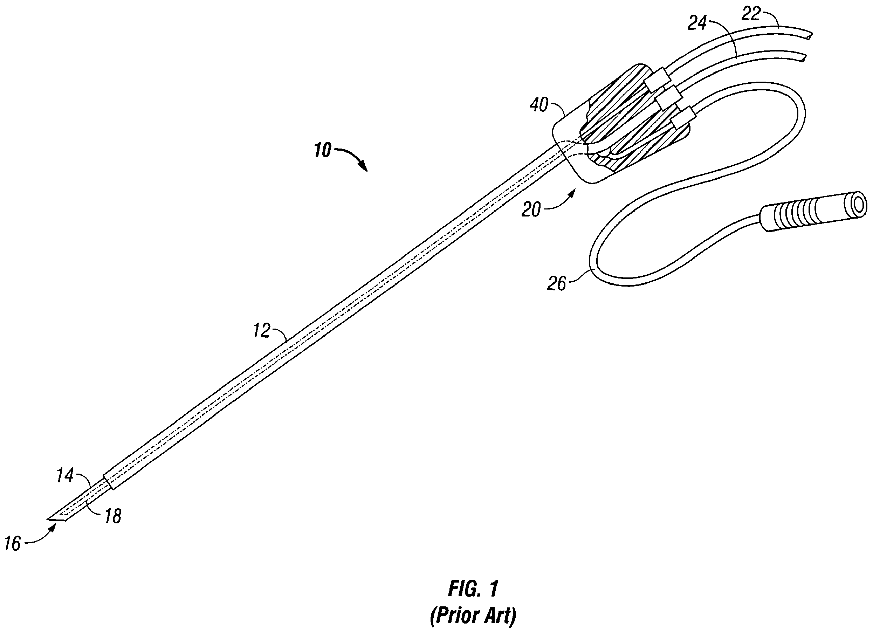

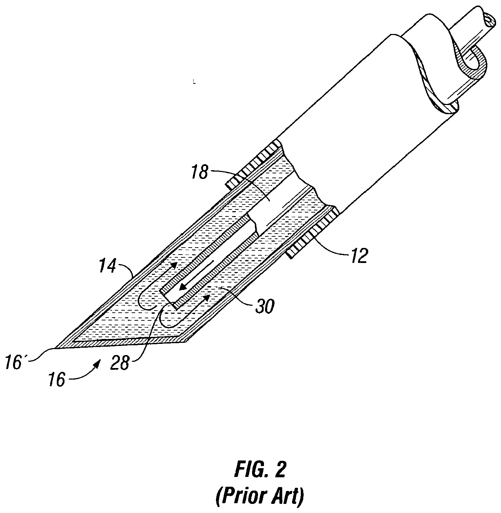

Referring initially to FIGS. 1 and 2, a prior art needle electrode according is shown and described and is generally designated as 10. As seen in FIG. 1, needle electrode 10 includes a distal end 16 and a proximal end 20 and further includes an outer tube 14 having a tip part 16 which is exposed and a tip point 16' (see FIG. 2) which is construed so as to penetrate tissue with a minimum risk of hemorrhage from the puncture tract. The non-exposed part of the outer tube 14 is surrounded by an insulating material 12. A distal portion of outer tube 14 is non-insulated and thereby exposed for DC or AC, preferably RF delivery. An inner tube 18 is provided inside the tube 14 co-axially with the outer tube 14.

An adapter 40 is provided at the proximal end 20 of needle electrode 10, opposite the tip part or distal end 16. The adapter 40 is equipped with a line 22, the line 22 being connected to the inner tube 18 and communicating therewith for providing a cooling fluid, such as water, to the distal end 16 of needle electrode 10. The water is led through the inner tube 18 to the tip part 16 and away from the tip part through the interior of the outer tube 14. The outer tube 14 is connected to and communicates with a line 24 for discharge of the cooling water. Lines 22 and 24 each communicate with a cooling water reservoir (not shown). Circulation of the cooling water is established with a pump (not shown). The outer tube 14 of the cooled needle electrode 10 is connected to a RF electrosurgical generator (not shown) through line 26 for providing power to the cooled needle electrode 10.

In FIG. 2, the tip part or distal end 16 of the cooled needle electrode 10 of FIG. 1 is shown. As seen in FIG. 2, the cooling water flows through the inner tube 18 and out at a tip 28 of the inner tube 18 and flows into the tip part 16 and out of the outer tube 14 shown at 30 for thereby providing a cooled needle electrode 10.

Preferred embodiments of the presently disclosed ablation system will now be described in detail with reference to the drawing figures wherein like reference numerals identify similar or identical elements. As used herein, the term "distal" refers to that portion which is further from the user while the term "proximal" refers to that portion which is closer to the user.

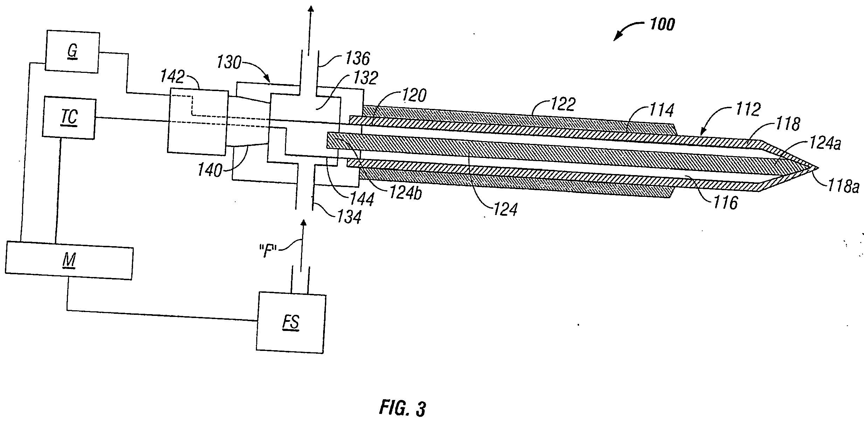

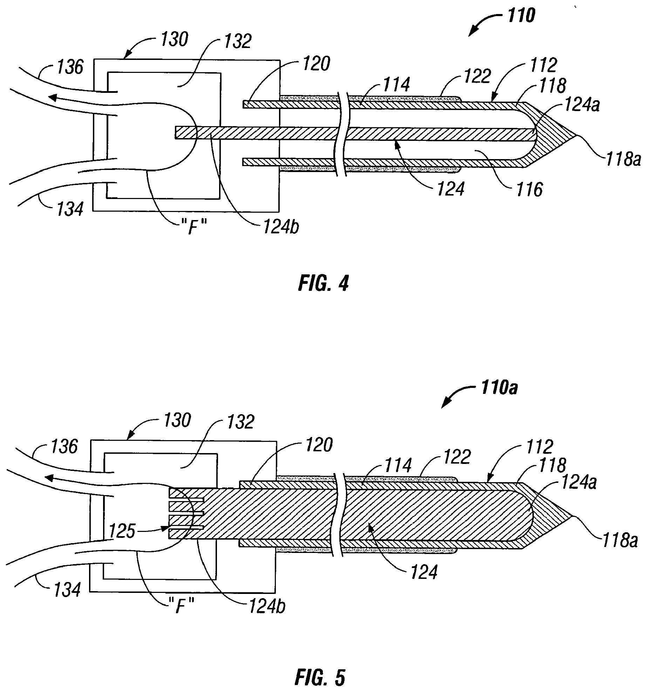

Referring now to FIGS. 3 and 4, an ablation system, in accordance with an embodiment of the present disclosure, is shown generally as 100. Ablation system 100 includes an ablation electrode assembly 110 operatively connected to an electrosurgical energy source "G" (e.g., an electrosurgical generator), and a source of cooling fluid "FS". A microprocessor or computer "M" may be connected to energy source "G" and fluid source "FS" for controlling and monitoring the operating parameters of ablation system 100.

As seen in FIGS. 3 and 4, ablation electrode assembly 110 includes an elongate ablation needle 112 which is configured and dimensioned for insertion into a patient, either percutaneously or intraoperatively. Ablation needle 112 includes a substantially cylindrical body or shaft portion 114 defining a cavity or chamber 116 therein. Ablation needle 112 includes a distal end portion 118 having a sharpened tip 118a, and a proximal end portion 120 configured and adapted for connection to a hub 130 or the like. Desirably, ablation needle 112 is fabricated from electrically conductive material, such as, for example, stainless steel, titanium, etc.

Ablation electrode assembly 110 has an insulative coating 122 over at least a portion of the length of ablation needle 112, preferably, over most of the length of ablation needle 112. Desirably, insulative coating 122 extends from hub 130 to distal end portion 118 of ablation needle 112, such that distal end portion 118 of ablation needle 112 is exposed or un-insulated. Insulative coating 122 selectively prevents the flow of electrical current from shaft portion 114 of ablation needle 112 into surrounding tissue. Thus, insulative coating 122 shields the intervening tissue from RF current, so that such tissue is not substantially heated along the length of shaft portion 114 except by the heating effect from distal end portion 118 which is exposed.

Ablation electrode assembly 110 further includes at least one heat sink, in the form of heat strap or heat pipe 124 extending through cavity 116 of ablation needle 112. While a single heat strap 124 is shown and will be described, it is envisioned and within the scope of the present disclosure for a plurality of heat straps 124 to be provided. Heat strap 124 includes a distal end 124a operatively secured to ablation needle 112 and a proximal end 124b extending into a cavity 132 formed in hub 130. In the present embodiment, distal end 124a of heat strap 124 is operatively connected or secured to distal end portion 118 of ablation needle 112. In an embodiment, distal end 124a of heat strap 124 is bonded to distal end portion 118 of ablation needle 112 with a thermally conductive adhesive or the like.

Heat strap 124 is fabricated from a highly heat conductive anisotropic material, such as, for example, graphite fiber. Accordingly, in use, as will be described in greater detail below, heat strap 124 draws heat away from distal end portion 118 of ablation needle 112 and dissipates the heat along a length thereof. In order to increase the efficiency and the rate of heat dissipation, as will be described in greater detail below, a cooling fluid may be circulated over proximal end 124b of heat strap 124.

As seen in FIG. 3, ablation system 100 further includes a hub 130 configured and adapted to support ablation electrode assembly 110. Hub 130 defines a chamber 132 therein, an inlet conduit 134 for delivering cooling fluid "F" into chamber 132 from fluid source "FS", and an outlet conduit 136 for delivering cooling fluid "F" from chamber 132. In operation, cooling fluid "F" is communicated into chamber 132 through inlet conduit 134 and out of chamber 132 through outlet conduit 136.

As mentioned above, with proximal end 124b of heat strap 124 extending into chamber 132 of hub 130, as cooling fluid "F" is circulated through chamber 132 of hub 130, heat or energy is withdrawn from proximal end 124b of heat strap 124 and carried away to fluid source "FS" for re-cooling and the like.

As seen in FIG. 3, hub 130 may include a proximal connector known as a luer connector, which is a tapered hole 140 or the like. Into female luer connector 140, a hub of a high frequency or thermo-sensing electrode 142 may be inserted and sealed by its male luer connection. A probe 144 of thermo-sensing electrode 142 may be connected to ablation needle 112 which can sense the temperature of ablation needle 112 at that point, or alternatively, may sense the temperature of distal end portion 118. Since distal end portion 118 of ablation needle 112 is contiguous and in contact on its external surface with the target tissue within the patient's body, thermo-sensing probe 144 can, depending on the thermal contact with ablation needle 112, get a measure of the temperature of the tissue immediately outside of distal end portion 118.

Connected to or within the hub of the high frequency and/or thermo-sensing electrode 142 are connections indicated by the dashed lines which connect to a high frequency electrosurgical generator "G" and/or a thermal-sensing circuit "TC" that may be outside of the body.

Electrosurgical generator "G" may be the source of high frequency voltage which produces the high frequency current that emanates from the distal end portion 118 of ablation needle 112. The thermal-sensing circuit "TC" may be of a thermocouple type and the temperature sensor could also be a bi-metal junction thermocouple such as a copper constantan.

Turning now to FIG. 5, an alternate embodiment of ablation electrode assembly is generally shown as 110a. Ablation electrode assembly 110a is substantially similar to ablation electrode assembly 110 and thus will only be discussed in detail to the extent necessary to identify differences in construction and/or operation. As seen in FIG. 5, heat strap 124 completely fills cavity 116 of ablation needle 112. In so doing, dissipation of heat and/or energy may take place along substantially the entire length of ablation needle 112.

As mentioned above with regard to ablation electrode assembly 110, with regard to ablation electrode assembly 110a, with proximal end 124b of heat strap 124 extending into chamber 132 of hub 130, as cooling fluid "F" is circulated through chamber 132 of hub 130, heat or energy is withdrawn from proximal end 124b of heat strap 124 and carried away to fluid source "FS" for re-cooling and the like. It is contemplated that proximal end 124b of heat strap 124 may include a plurality of fingers 125 or the like, thereby increasing the surface area over which fluid "F" is circulated and thus increasing the rate of heat and/or energy dissipation.

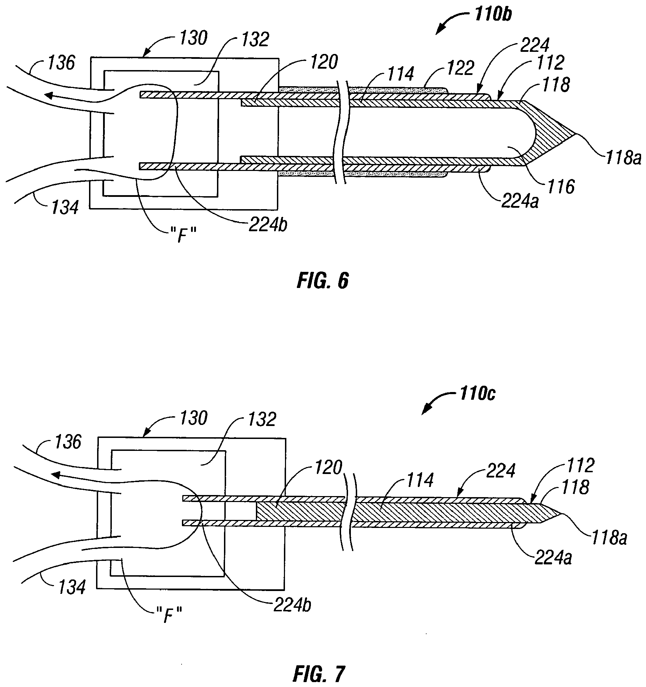

Turning now to FIGS. 6 and 7, alternate embodiments of ablation electrode assemblies are generally shown as 110b and 110c, respectively. Ablation electrode assemblies 110b, 110c are substantially similar to ablation electrode assembly 110 and thus will only be discussed in detail to the extent necessary to identify differences in construction and/or operation.

As seen in FIG. 6, ablation electrode assembly 110b includes a heat sink or heat strap, in the form of a sleeve or coating 224 wrapped around or surrounding at least a portion of the length of ablation needle 112, preferably over most of the length of ablation needle 112. Desirably, heat strap 224 extends to and not beyond distal end portion 118 of ablation needle 112, thus maintaining distal end portion 118 of ablation needle 112 exposed. Heat strap 224 includes a proximal end portion 224b which extends through hub 130 and into cavity 132.

In this embodiment, insulating coating 122 desirably encases and/or surrounds substantially all of heat strap 224. Alternatively, heat strap 224 may function as an insulating sleeve or barrier, thus eliminating the need for an insulating coating 122 disposed on or about heat strap 224.

As seen in FIG. 7, ablation electrode assembly 110c may include an ablation needle 112 which is solid (i.e., no cavity 116 is provided). In the present embodiment, heat strap 224 substantially encases ablation needle 112. Desirably, distal end portion 118 of ablation needle 112 remains exposed. Heat strap 224 includes a proximal end portion 224b which extends through hub 130 and into cavity 132. As with the embodiment in FIG. 6, heat strap 224 of the present embodiment also functions as an insulating coating or the like.

Desirably, distal end portion 118 of ablation needle 112 is exposed about 2.0 cm in length. Ablation needle 112 desirably has a transverse diameter of about 2 mm.

In operation, ablation electrode assembly 110 is inserted into an operative site of a patient, either percutaneously or intra-operatively. Desirably, ablation electrode assembly 110 is inserted into the operative site until distal end portion 118 of ablation needle 112 is positioned or disposed adjacent to or within a target tissue to be ablated. A return pad or return electrode (not shown) may now be or may previously have been operatively adhered to or connected to the patient. Any known technique may be used to visually position distal end portion 118 of ablation needle 112 in the operative site, such as, for example and not limited to, X-ray imaging, CT scanning, MRI's, fluoroscopy, angiographic, PET, SPECT, MEG, ultrasonic imaging, etc.

With distal end portion 118 of ablation needle 112 in position, electrosurgical energy is delivered from electrosurgical generator "G" to distal end portion 118 of ablation needle 112. Desirably, an effective amount of electrosurgical energy at an effective energy level and for an effective duration of time is delivered to distal end portion 118 of ablation needle 112 to treat and/or ablate the target tissue of the like. For example, electrosurgical generator "G" may deliver an energy frequency of from about 100 kilo Hertz to several hundred mega Hertz. An example of an electrosurgical generator "G" capable of producing such an output is the lesion generator available from Radionics, Inc, of Burlington, Mass.

Either prior to or simultaneously with the delivery of electrosurgical energy to distal end portion 118 of ablation needle 112, a fluid "F" (e.g., water, saline, etc.) is circulated through chamber 132 of hub 130. Desirably, fluid "F" is cooled to a temperature of about 0° C prior to circulation. During circulation, fluid "F" enters chamber 132 of hub 130 through inlet conduit 134 and exits chamber 132 of hub 130 through outlet conduit 136. In so doing, fluid "F" contacts and/or washes over/across proximal end 124b or 224b of heat straps 124, 224, respectively, and withdraws heat and/or energy therefrom and, in turn, from ablation needle 112.

Following treatment or ablation of the target tissue, ablation electrode assembly 110 may be withdrawn from the target site and re-introduced into another target site, into the same target site from a different angle or approach, or in substantially the same location.

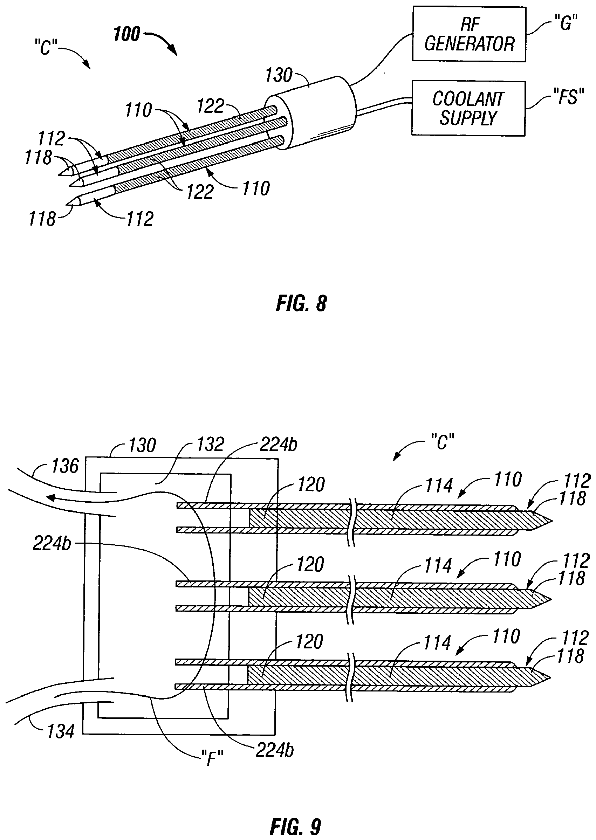

Turning now to FIGS. 8 and 9, ablation system 100 may include a cluster "C" or plurality of ablation electrode assemblies 110 supported in hub 130. Desirably, any of ablation electrode assemblies 110-110c may be supported on or operatively connected to hub 130. Cluster "C" of ablation electrode assemblies 110 are each connected to electrosurgical generator "G". Accordingly, cluster "C" will effectively act as a larger electrode.

It is envisioned that ablation electrode assemblies 110 may be arranged in a substantially linear array, as shown in FIG. 9, or may be evenly spaced from one another, as shown in FIG. 8. While three ablation electrode assemblies 110 are shown and described, it is envisioned that any number of ablation electrode assemblies may be provided.

In use, as fluid "F" is circulated through chamber 132 of hub 130, fluid "F" circulates over or washes across proximal ends 224b of heat straps 224 of each ablation electrode assembly 110 extending into chamber 132 of hub 130. In so doing, heat and/or energy is/are drawn from each heat strap 224 and, in turn, from each ablation needle 112.

The use of a multiplicity of N ablation electrode assemblies 110 increases the overall conductive exposed tip area by which to send RF current for heating into the target tissue site. This increases the heating power that may be delivered and thus increases the size of the ablation volume possible.

The cooling capacity of a multiplicity ofN ablation electrode assemblies also increases as the number N increases. Increasing the number of ablation electrode assemblies increases the cooling surface area near cluster "C". Thus, the heat sinking effect from a cluster of ablation electrode assemblies is greater than the heat sinking effect from a single ablation electrode assembly. This allows the size of a lesion to be expanded accordingly.

For example, in specific embodiments, ablation electrode assemblies 110 of cluster "C" may have diameters in the range of about 0.5 mm to about 3.0 mm. An advantage of a multiplicity of coherent smaller electrodes versus insertion of a single large electrode is that the smaller electrodes will produce less chance of hemorrhage.

Although the subject device, systems and methods have been described with respect to preferred embodiments, it will be readily apparent, to those having ordinary skill in the art to which it appertains, that changes and modifications may be made thereto without departing from the scope of the subject of the present disclosure.

## Claims

1. An ablation electrode assembly (10) operatively connectable to a source of electrosurgical energy and to a source of cooling fluid, the ablation electrode assembly comprising:
a hub (130) defining a chamber (132) therein;
at least one electrically conductive ablation needle (112) extending from the hub, the needle being cylindrical and defining cavity (116) therein, wherein the cavity of the ablation needle extends to the distal end portion of the needle and the ablation needle including a distal end portion (118) configured to penetrate tissue, said distal end portion of the needle being electrically and thermally conductive for establishing electric and thermal communication with the tissue;
a heat sink (124) fabricated from an anisotropic material disposed within the cavity operatively connected to the ablation needle, wherein a distal end of the heat sink is in conductive engagement with a distal end surface of the cavity of the ablation needle, the heat sink being connected to the ablation needle to draw energy away from at least the distal end portion thereof, the heat sink including a proximal end (124b) extending into the chamber of the hub;
an inlet conduit (134) fluidly connected to the hub for delivering fluid into the chamber thereof from the source of fluid, wherein the fluid withdraws energy from the proximal end of the heat sink,
an outlet conduit (136) fluidly connected to the chamber of the hub for delivering fluid from the chamber thereof,
, such that fluid enters the chamber of the hub through the inlet conduit and exits the chamber of the hub through the outlet conduit, whereby fluid contacts and washes over or across the proximal end of the heat sink and withdraws heat and/or energy therefrom and, in turn, from the ablation needle.

2. The ablation electrode assembly according to claim 1, wherein the heat sink is fabricated from a conductive material.

3. The ablation electrode assembly according to claim 1 or 2, wherein the heat sink is fabricated from a graphite fiber.

4. The ablation electrode assembly according to any one of the preceding claims, further comprising an insulative coating (122) surrounding at least a portion of a length of the ablation needle.

5. The ablation electrode assembly according to claim 4, wherein the distal end portion of the ablation needle is exposed.

6. The ablation electrode assembly according to any one of the preceding claims, wherein the distal end portion of the ablation needle has a sharpened tip (118a).

7. The ablation electrode assembly according to any one of claims 1 to 6, wherein the heat sink is in the form of a heat strap extending through the cavity of the ablation needle, wherein a distal end (124a) of the heat strap is secured to the distal end portion of the ablation needle.

8. The ablation electrode assembly of claim 7, wherein the distal end of the heat strap is bonded to the distal end portion of the ablation needle with a thermally conductive adhesive.

9. An ablation electrode assembly (110) operatively connectable to a source of electrosurgical energy and to a source of cooling fluid, the ablation electrode assembly comprising:
a hub (130) defining a chamber (132) therein;
at least one electrically conductive ablation needle (112) extending from the hub, the ablation needle including a distal end portion (118) configured to penetrate tissue, said distal end portion being electrically and thermally conductive for establishing electric and thermal communication with the tissue;
a heat sink (224) which encases at least a portion of a length of the ablation needle to draw energy away from at least the distal end portion thereof, the heat sink including a proximal end (224b) extending into the chamber of the hub;
an inlet conduit (134) fluidly connected to the hub for delivering fluid into the chamber thereof from the source of fluid, wherein the fluid withdraws energy from the proximal end of the heat sink,
an outlet conduit (136) fluidly connected to the chamber of the hub for delivering fluid from the chamber thereof,
such that fluid enters the chamber of the hub through the inlet conduit and exits the chamber of the hub through the outlet conduit, whereby fluid contacts and washes over or across the proximal end of the heat sink and withdraws heat and/or energy therefrom and, in turn, from the ablation needle.

10. The ablation electrode assembly according to claim 9, wherein the distal end portion of the ablation needle is exposed.

11. The ablation electrode assembly according to claim 9 or 10, further comprising an insulative coating (122) surrounding at least a portion of a length of the heat sink encasing the ablation needle.

12. The ablation electrode assembly of claim 9, 10 or 11, wherein the heat sink is in the form of a sleeve (224) surrounding the ablation needle over most of the length of the ablation needle.

13. The ablation electrode assembly of claim 9, 10, 11 or 12, wherein the ablation needle is solid.

14. The ablation electrode assembly according to any preceding claim, further comprising a plurality of ablation needles (110).

15. An ablation system comprising:
the ablation electrode assembly according to any one of the preceding claims;
a source of electrosurgical energy (G) electrically connected to the ablation needle; and
a source of cooling fluid (FS) fluidly connected to the chamber of the hub by the conduits.

16. The ablation system according to claim 15, further comprising a thermal-sensing circuit (TC) electrically connected to the ablation needle for measuring a temperature of the ablation needle.

17. The ablation system according to any one of the preceding claims, 15 or 16 further comprising a microprocessor (M) connected to and for coordinating operation of the source of electrosurgical energy and the source of fluid.

## Patentansprüche

1. Eine Ablationselektrodenanordnung (10), die mit einer Quelle von elektrochirurgischer Energie und mit einer Quelle von Kühlfluid operativ verbunden werden kann, wobei die Ablationselektrodenanordnung aufweist:
eine Büchse (130), die eine Kammer (132) darin definiert;
wenigstens eine elektrisch leitende Ablationsnadel (112), die sich von der Büchse erstreckt, wobei die Nadel zylindrisch ist und einen Hohlraum (116) darin definiert, wobei sich der Hohlraum von der Ablationsnadel zu dem distalen Endabschnitt von der Nadel erstreckt und die Ablationsnadel einen distalen Endabschnitt (118) umfasst, der dazu konfiguriert ist, Gewebe zu durchdringen, wobei der besagte distale Endabschnitt von der Nadel zum Aufbauen einer elektrischen und thermischen Verbindung mit dem Gewebe elektrisch und thermisch leitend ist;
einen Kühlkörper (124), der aus einem anisotropen, in dem Hohlraum angeordneten Material hergestellt ist, operativ mit der Ablationsnadel verbunden, wobei ein distales Ende von dem Kühlkörper in leitendem Eingriff mit einer distalen Endfläche von dem Hohlraum von der Ablationsnadel steht, wobei der Kühlkörper mit der Ablationsnadel verbunden ist, um Energie von wenigstens dem distalen Endabschnitt davon weg zu ziehen, wobei der Kühlkörper ein proximales Ende (124b) umfasst, das sich in die Kammer von der Büchse hinein erstreckt;
eine Einlassleitung (134), die mit der Büchse fluidverbunden ist, um Fluid von der Quelle von Fluid in die Kammer davon zu fördern, wobei das Fluid Energie aus dem proximalen Ende von dem Kühlkörper entzieht;
eine Auslassleitung (136), die mit der Kammer von der Büchse fluidverbunden ist, um Fluid aus der Kammer davon zu fördern,
so dass Fluid durch die Einlassleitung in die Kammer von der Büchse eintritt und die Kammer von der Büchse durch die Auslassleitung verlässt, wobei Fluid das proximale Ende von dem Kühlkörper kontaktiert und dieses überspült oder durchspült und Wärme und/oder Energie davon und wiederum von der Ablationsnadel entzieht.

2. Die Ablationselektrodenanordnung nach Anspruch 1, wobei der Kühlkörper aus einem leitenden Material hergestellt ist.

3. Die Ablationselektrodenanordnung nach Anspruch 1 oder 2, wobei der Kühlkörper aus Graphitfaser hergestellt ist.

4. Die Ablationselektrodenanordnung nach irgendeinem der vorangegangenen Ansprüche, weiter eine isolierende Beschichtung (122) aufweisend, die wenigstens einen Abschnitt von einer Länge von der Ablationsnadel umgibt.

5. Die Ablationselektrodenanordnung nach Anspruch 4, wobei der distale Endabschnitt von der Ablationsnadel exponiert ist.

6. Die Ablationselektrodenanordnung nach irgendeinem der vorangegangenen Ansprüche, wobei der distale Endabschnitt von der Ablationsnadel eine geschärfte Spitze (118a) hat.

7. Die Ablationselektrodenanordnung nach irgendeinem der Ansprüche 1 bis 6, wobei der Kühlkörper in der Form von einem Kühlbügel ist, der sich durch den Hohlraum von der Ablationsnadel erstreckt, wobei ein distales Ende (124a) von dem Kühlbügel an dem distalen Endabschnitt von der Ablationsnadel gesichert ist.

8. Die Ablationselektrodenanordnung nach Anspruch 7, wobei das distale Ende von dem Kühlbügel mit einem thermisch leitenden Klebstoff mit dem distalen Endabschnitt von der Ablationsnadel verbunden ist.

9. Eine Ablationselektrodenanordnung (110), die mit einer Quelle von elektrochirurgischer Energie und mit einer Quelle von Kühlfluid operativ verbunden werden kann, wobei die Ablationselektrodenanordnung aufweist:
eine Büchse (130), die eine Kammer (132) darin definiert;
wenigstens eine elektrisch leitende Ablationsnadel (112), die sich von der Büchse erstreckt, wobei die Ablationsnadel einen distalen Endabschnitt (118) umfasst, der dazu konfiguriert ist, Gewebe zu durchdringen, wobei der besagte distale Endabschnitt zum Aufbauen einer elektrischen und thermischen Verbindung mit dem Gewebe elektrisch und thermisch leitend ist;
einen Kühlkörper (224), der wenigstens einen Abschnitt von einer Länge von der Ablationsnadel ummantelt, um Energie von wenigstens dem distalen Endabschnitt davon weg zu ziehen, wobei der Kühlkörper ein proximales Ende (224b) umfasst, das sich in die Kammer von der Büchse hinein erstreckt;
eine Einlassleitung (134), die mit der Büchse fluidverbunden ist, um Fluid von der Quelle von Fluid in die Kammer davon zu fördern, wobei das Fluid Energie aus dem proximalen Ende von dem Kühlkörper entzieht;
eine Auslassleitung (136), die mit der Kammer von der Büchse fluidverbunden ist, um Fluid aus der Kammer davon zu fördern,
so dass Fluid durch die Einlassleitung in die Kammer von der Büchse eintritt und die Kammer von der Büchse durch die Auslassleitung verlässt, wobei Fluid das proximale Ende von dem Kühlkörper kontaktiert und dieses überspült oder durchspült und Wärme und/oder Energie davon und wiederum von der Ablationsnadel entzieht.

10. Die Ablationselektrodenanordnung nach Anspruch 9, wobei der distale Endabschnitt von der Ablationsnadel exponiert ist.

11. Die Ablationselektrodenanordnung nach Anspruch 9 oder 10, weiter eine isolierende Beschichtung (122) aufweisend, die wenigstens einen Abschnitt von einer Länge von dem Kühlkörper umgibt, der die Ablationsnadel ummantelt.

12. Die Ablationselektrodenanordnung nach Anspruch 9, 10 oder 11, wobei der Kühlkörper in der Form von einer Hülle (224) ist, die die Ablationsnadel über den größten Teil von der Länge von der Ablationsnadel umgibt.

13. Die Ablationselektrodenanordnung nach Anspruch 9, 10, 11 oder 12, wobei die Ablationsnadel solide ist.

14. Die Ablationselektrodenanordnung nach einem vorangegangen Anspruch, weiter eine Vielzahl von Ablationsnadeln (110) aufweisend.

15. Ein Ablationssystem, aufweisend:
die Ablationselektrodenanordnung nach irgendeinem der vorangegangenen Ansprüche;
eine Quelle von elektrochirurgischer Energie (G), die mit der Ablationsnadel elektrisch verbunden ist; und
eine Quelle von Kühlfluid (FS), die mit der Kammer von der Büchse durch die Leitungen fluidverbunden ist.

16. Das Ablationssystem nach Anspruch 15, weiter einen thermischen Sensorkreis (TC) aufweisend, der mit der Ablationsnadel elektrisch verbunden ist, um eine Temperatur von der Ablationsnadel zu messen.

17. Das Ablationssystem nach irgendeinem der vorangegangen Ansprüche 15 oder 16, weiter einen Mikroprozessor (M) aufweisend, der mit der Quelle von elektrochirurgischer Energie und der Quelle von Fluid verbunden und zum Koordinieren eines Betriebs davon ist.

## Revendications

1. Ensemble formant électrode d'ablation (10) pouvant être relié de manière opérationnelle à une source d'énergie électrochirurgicale et à une source de fluide de refroidissement, l'ensemble formant électrode d'ablation comprenant :
un moyeu (130) définissant une chambre (132) en son sein ;
au moins une aiguille d'ablation électriquement conductrice (112) s'étendant à partir du moyeu, l'aiguille étant cylindrique et définissant une cavité (116) en son sein, dans laquelle la cavité de l'aiguille d'ablation s'étend jusqu'à la partie d'extrémité distale de l'aiguille et l'aiguille d'ablation incluant une partie d'extrémité distale (118) configurée pour pénétrer un tissu, ladite partie d'extrémité distale de l'aiguille étant électriquement et thermiquement conductrice pour établir une communication électrique et thermique avec le tissu ;
un puits de chaleur (124) fabriqué à partir d'une matière anisotrope disposée à l'intérieur de la cavité relié de manière opérationnelle à l'aiguille d'ablation, dans lequel une extrémité distale du puits de chaleur est en prise conductrice avec une surface d'extrémité distale de la cavité de l'aiguille d'ablation, le puits de chaleur étant relié à l'aiguille d'ablation pour extraire l'énergie au moins à partir de son extrémité distale, le puits de chaleur incluant une extrémité proximale (124b) s'étendant dans la chambre du moyeu ;
un conduit d'entrée (134) relié à fluide au moyeu pour délivrer du fluide dans la chambre de ce dernier en provenance de la source de fluide, dans lequel le fluide retire de l'énergie à partir de l'extrémité proximale du puits de chaleur,
un conduit de sortie (136) relié à fluide à la chambre du moyeu pour délivrer du fluide à partir de la chambre de ce dernier,
de telle sorte que du fluide pénètre dans la chambre du moyeu à travers le conduit d'entrée et quitte la chambre du moyeu à travers le conduit de sortie, de sorte que du fluide entre en contact et submerge ou traverse l'extrémité proximale du puits de chaleur et retire la chaleur et/ou l'énergie de ce dernier et, à son tour, de l'aiguille d'ablation.

2. Ensemble formant électrode d'ablation selon la revendication 1, dans lequel le puits de chaleur est fabriqué à partir d'une matière conductrice.

3. Ensemble formant électrode d'ablation selon la revendication 1 ou 2, dans lequel le puits de chaleur est fabriqué à partir d'une fibre de graphite.

4. Ensemble formant électrode d'ablation selon l'une quelconque des revendications précédentes, comprenant en outre un revêtement isolant (122) entourant au moins une partie d'une longueur de l'aiguille d'ablation.

5. Ensemble formant électrode d'ablation selon la revendication 4, dans lequel la partie d'extrémité distale de l'aiguille d'ablation est exposée.

6. Ensemble formant électrode d'ablation selon l'une quelconque des revendications précédentes, dans lequel la partie d'extrémité distale de l'aiguille d'ablation a une pointe aiguisée (118a).

7. Ensemble formant électrode d'ablation selon l'une quelconque des revendications 1 à 6, dans lequel le puits de chaleur est sous la forme d'une bande thermique s'étendant à travers la cavité de l'aiguille d'ablation, dans lequel une extrémité distale (124a) de la bande thermique est fixée à la partie d'extrémité distale de l'aiguille d'ablation.

8. Ensemble formant électrode d'ablation selon la revendication 7, dans lequel l'extrémité distale de la bande thermique est liée à la partie d'extrémité distale de l'aiguille d'ablation par un adhésif thermiquement conducteur.

9. Ensemble formant électrode d'ablation (110) pouvant être relié de manière opérationnelle à une source d'énergie électrochirurgicale et à une source de fluide de refroidissement, l'ensemble formant électrode d'ablation comprenant :
un moyeu (130) définissant une chambre (132) en son sein ;
au moins une aiguille d'ablation électriquement conductrice (112) s'étendant à partir du moyeu, l'aiguille d'ablation incluant une partie d'extrémité distale (118) configurée pour pénétrer dans un tissu, ladite partie d'extrémité distale étant électriquement et thermiquement conductrice pour établir une communication électrique et thermique avec le tissu ;
un puits de chaleur (224) qui englobe au moins une partie d'une longueur de l'aiguille d'ablation pour extraire l'énergie au moins à partir de la partie d'extrémité distale de celle-ci, le puits de chaleur incluant une extrémité proximale (224b) s'étendant dans la chambre du moyeu ;
un conduit d'entrée (134) relié à fluide au moyeu pour délivrer du fluide dans la chambre de ce dernier en provenance de la source de fluide, dans lequel le fluide retire de l'énergie à partir de l'extrémité proximale du puits de chaleur,
un conduit de sortie (136) relié à fluide à la chambre du moyeu pour délivrer du fluide à partir de la chambre de ce dernier,
de telle sorte que du fluide pénètre dans la chambre du moyeu à travers le conduit d'entrée et quitte la chambre du moyeu à travers le conduit de sortie, de sorte que du fluide entre en contact et submerge ou traverse l'extrémité proximale du puits de chaleur et retire la chaleur et/ou l'énergie de ce dernier et, à son tour, de l'aiguille d'ablation.

10. Ensemble formant électrode d'ablation selon la revendication 9, dans lequel la partie d'extrémité distale de l'aiguille d'ablation est exposée.

11. Ensemble formant électrode d'ablation selon la revendication 9 ou 10, comprenant en outre un revêtement isolant (122) entourant au moins une partie d'une longueur du puits de chaleur englobant l'aiguille d'ablation.

12. Ensemble formant électrode d'ablation selon la revendication 9, 10 ou 11, dans lequel le puits de chaleur est sous la forme d'un manchon (224) entourant l'aiguille d'ablation sur la plupart de la longueur de l'aiguille d'ablation.

13. Ensemble formant électrode d'ablation selon la revendication 9, 10, 11 ou 12, dans lequel l'aiguille d'ablation est pleine.

14. Ensemble formant électrode d'ablation selon l'une quelconque des revendications précédentes, comprenant en outre une pluralité d'aiguilles d'ablation (110).

15. Système d'ablation comprenant :
l'ensemble formant électrode d'ablation selon l'une quelconque des revendications précédentes ;
une source d'énergie électrochirurgicale (G) électriquement reliée à l'aiguille d'ablation ; et
une source de fluide de refroidissement (FS) reliée à fluide à la chambre du moyeu par les conduits.

16. Système d'ablation selon la revendication 15, comprenant en outre un circuit de détection thermique (TC) électriquement relié à l'aiguille d'ablation pour mesurer une température de l'aiguille d'ablation.

17. Système d'ablation selon l'une quelconque des revendications 15 ou 16, comprenant en outre un microprocesseur (M) relié à la source d'énergie électrochirurgicale et à la source de fluide et pour coordonner leur fonctionnement.
